# EUROPEAN PATENT APPLICATION

(11) **EP 1 647 292 A1**
(43) Date of publication of application: **19.04.2006**
(21) Application number: 04730727.7
(22) Date of filing: 30.04.2004
(51) Int. Cl.: A61M 5/30, C12N 15/00

(54) **NEEDLELESS SYRINGE HAVING MEDICAL AGENT ACCOMMODATED THEREIN**

(30) Priority: 09.05.2003 JP 2003131126
(71) Applicant: Morishita, Ryuichi, Suita-shi, Osaka 532-0003 (JP); AnGes MG, Inc., Ibaraki-shi, Osaka 567-0085 (JP); SHIMADZU CORPORATION, Kyoto-shi, Kyoto 604-8511 (JP)
(72) Inventor: MORISHITA, Ryuichi, Suita-shi, Osaka 532-0003 (JP); TOMITA, Naruya, Okayama-shi, Okayama 700-0951 (JP); KUNUGIZA, Yasuo, Osaka 565-0873 (JP); TANIYAMA, Yoshiaki, Suita-shi, Osaka 565-0821 (JP); KOIKE, Hiromi, Nishinomiya-shi, Hyogo 662-0934 (JP); UDAGAWA, Haruhide c/o Shimadzu Corporation, Kyoto-shi, Kyoto 604-8511 (JP); KIMURA, Tatsuo c/o Shimadzu Corporation, Kyoto-shi, Kyoto 604-8511 (JP)
(74) Representative: Pohlmann, Eckart
(86) International application number: PCT/JP2004/006343
(87) International publication number: WO 2004/110533

(57) **Abstract**

The present invention provides a needleless syringe having, accommodated therein, a pharmaceutical preparation containing genes and/or analogues thereof. A needleless syringe comprising: a medical agent chamber 22 having one or multiple nozzle bore(s) 25 and accommodated with a medical agent L, and a syringe main body 12 being provided with pressure application means 32 to apply pressure on the medical agent L toward a direction of the nozzle bore (s) 25 and effect emission of the medical agent L through the nozzle bore (s) 25, wherein the medical agent L is a pharmaceutical preparation containing genes and/or analogues thereof. Examples of the medical agent L include pharmaceutical preparation containing HGF gene and pharmaceutical preparation containing NF-κB decoy oligonucleotide.

SEQUENCE LISTING

<110> Morishita, Ryuuichi; AnGes MG, Inc.; Shimadzu Corp.

<120> Needleless Syringe Having Medical Agent Accommodated Therein

<130> G16-13PCT

<150> JP 2003-131126

<151> 2003-05-09

<160> 3

<170> PatentIn Ver. 2.1

<210> 1

<211> 728

<212> PRT

<213> Homo Sapiens

<400> 1

Met Trp Val Thr Lys Leu Leu Pro Ala Leu Leu Leu Gln His Val Leu
 1               5                  10                  15

Leu His Leu Leu Leu Leu Pro Ile Ala Ile Pro Tyr Ala Glu Gly Gln
            20                  25                  30

Arg Lys Arg Arg Asn Thr Ile His Glu Phe Lys Lys Ser Ala Lys Thr
        35                  40                  45

Thr Leu Ile Lys Ile Asp Pro Ala Leu Lys Ile Lys Thr Lys Lys Val

    50                  55                  60
Asn Thr Ala Asp Gln Cys Ala Asn Arg Cys Thr Arg Asn Lys Gly Leu
65                  70                  75                  80
Pro Phe Thr Cys Lys Ala Phe Val Phe Asp Lys Ala Arg Lys Gln Cys
                85                  90                  95
Leu Trp Phe Pro Phe Asn Ser Met Ser Ser Gly Val Lys Lys Glu Phe
            100                 105                 110
Gly His Glu Phe Asp Leu Tyr Glu Asn Lys Asp Tyr Ile Arg Asn Cys
        115                 120                 125
Ile Ile Gly Lys Gly Arg Ser Tyr Lys Gly Thr Val Ser Ile Thr Lys
    130                 135                 140
Ser Gly Ile Lys Cys Gln Pro Trp Ser Ser Met Ile Pro His Glu His
145                 150                 155                 160
Ser Phe Leu Pro Ser Ser Tyr Arg Gly Lys Asp Leu Gln Glu Asn Tyr
                165                 170                 175
Cys Arg Asn Pro Arg Gly Glu Glu Gly Gly Pro Trp Cys Phe Thr Ser
            180                 185                 190
Asn Pro Glu Val Arg Tyr Glu Val Cys Asp Ile Pro Gln Cys Ser Glu
        195                 200                 205
Val Glu Cys Met Thr Cys Asn Gly Glu Ser Tyr Arg Gly Leu Met Asp
    210                 215                 220
His Thr Glu Ser Gly Lys Ile Cys Gln Arg Trp Asp His Gln Thr Pro
225                 230                 235                 240
His Arg His Lys Phe Leu Pro Glu Arg Tyr Pro Asp Lys Gly Phe Asp
                245                 250                 255
Asp Asn Tyr Cys Arg Asn Pro Asp Gly Gln Pro Arg Pro Trp Cys Tyr

            260                 265                 270
Thr Leu Asp Pro His Thr Arg Trp Glu Tyr Cys Ala Ile Lys Thr Cys
        275                 280                 285
Ala Asp Asn Thr Met Asn Asp Thr Asp Val Pro Leu Glu Thr Thr Glu
    290                 295                 300
Cys Ile Gln Gly Gln Gly Glu Gly Tyr Arg Gly Thr Val Asn Thr Ile
305                 310                 315                 320
Trp Asn Gly Ile Pro Cys Gln Arg Trp Asp Ser Gln Tyr Pro His Glu
                325                 330                 335
His Asp Met Thr Pro Glu Asn Phe Lys Cys Lys Asp Leu Arg Glu Asn
            340                 345                 350
Tyr Cys Arg Asn Pro Asp Gly Ser Glu Ser Pro Trp Cys Phe Thr Thr
        355                 360                 365
Asp Pro Asn Ile Arg Val Gly Tyr Cys Ser Gln Ile Pro Asn Cys Asp
    370                 375                 380
Met Ser His Gly Gln Asp Cys Tyr Arg Gly Asn Gly Lys Asn Tyr Met
385                 390                 395                 400
Gly Asn Leu Ser Gln Thr Arg Ser Gly Leu Thr Cys Ser Met Trp Asp
                405                 410                 415
Lys Asn Met Glu Asp Leu His Arg His Ile Phe Trp Glu Pro Asp Ala
            420                 425                 430
Ser Lys Leu Asn Glu Asn Tyr Cys Arg Asn Pro Asp Asp Asp Ala His
        435                 440                 445
Gly Pro Trp Cys Tyr Thr Gly Asn Pro Leu Ile Pro Trp Asp Tyr Cys
    450                 455                 460
Pro Ile Ser Arg Cys Glu Gly Asp Thr Thr Pro Thr Ile Val Asn Leu

465                 470                 475                 480
Asp His Pro Val Ile Ser Cys Ala Lys Thr Lys Gln Leu Arg Val Val
                485                 490                 495
Asn Gly Ile Pro Thr Arg Thr Asn Ile Gly Trp Met Val Ser Leu Arg
            500                 505                 510
Tyr Arg Asn Lys His Ile Cys Gly Gly Ser Leu Ile Lys Glu Ser Trp
        515                 520                 525
Val Leu Thr Ala Arg Gln Cys Phe Pro Ser Arg Asp Leu Lys Asp Tyr
    530                 535                 540
Glu Ala Trp Leu Gly Ile His Asp Val His Gly Arg Gly Asp Glu Lys
545                 550                 555                 560
Cys Lys Gln Val Leu Asn Val Ser Gln Leu Val Tyr Gly Pro Glu Gly
                565                 570                 575
Ser Asp Leu Val Leu Met Lys Leu Ala Arg Pro Ala Val Leu Asp Asp
            580                 585                 590
Phe Val Ser Thr Ile Asp Leu Pro Asn Tyr Gly Cys Thr Ile Pro Glu
        595                 600                 605
Lys Thr Ser Cys Ser Val Tyr Gly Trp Gly Tyr Thr Gly Leu Ile Asn
    610                 615                 620
Tyr Asp Gly Leu Leu Arg Val Ala His Leu Tyr Ile Met Gly Asn Glu
625                 630                 635                 640
Lys Cys Ser Gln His His Arg Gly Lys Val Thr Leu Asn Glu Ser Glu
                645                 650                 655
Ile Cys Ala Gly Ala Glu Lys Ile Gly Ser Gly Pro Cys Glu Gly Asp
            660                 665                 670
Tyr Gly Gly Pro Leu Val Cys Glu Gln His Lys Met Arg Met Val Leu

        675                 680                 685
Gly Val Ile Val Pro Gly Arg Gly Cys Ala Ile Pro Asn Arg Pro Gly
    690                 695                 700
Ile Phe Val Arg Val Ala Tyr Tyr Ala Lys Trp Ile His Lys Ile Ile
705                 710                 715                 720
Leu Thr Tyr Lys Val Pro Gln Ser
                725

<210> 2
<211> 2187
<212> DNA
<213> Homo Sapiens

<400> 2
atgtgggtga ccaaactcct gccagccctg ctgctgcagc atgtcctcct gcatctcctc 60
ctgctcccca tcgccatccc ctatgcagag ggacaaagga aaagaagaaa tacaattcat 120
gaattcaaaa aatcagcaaa gactacccta atcaaaatag atccagcact gaagataaaa 180
accaaaaaag tgaatactgc agaccaatgt gctaatagat gtactaggaa taaaggactt 240
ccattcactt gcaaggcttt tgtttttgat aaagcaagaa aacaatgcct ctggttcccc 300
ttcaatagca tgtcaagtgg agtgaaaaaa gaatttggcc atgaatttga cctctatgaa 360
aacaaagact acattagaaa ctgcatcatt ggtaaaggac gcagctacaa gggaacagta 420
tctatcacta agagtggcat caaatgtcag ccctggagtt ccatgatacc acacgaacac 480
agctttttgc cttcgagcta tcggggtaaa gacctacagg aaaactactg tcgaaatcct 540
cgaggggaag aagggggacc ctggtgtttc acaagcaatc cagaggtacg ctacgaagtc 600
tgtgacattc ctcagtgttc agaagttgaa tgcatgacct gcaatgggga gagttatcga 660
ggtctcatgg atcatacaga atcaggcaag atttgtcagc gctgggatca tcagacacca 720

caccggcaca aattcttgcc tgaaagatat cccgacaagg gctttgatga taattattgc 780
cgcaatcccg atggccagcc gaggccatgg tgctatactc ttgaccctca cacccgctgg 840
gagtactgtg caattaaaac atgcgctgac aatactatga atgacactga tgttcctttg 900
gaaacaactg aatgcatcca aggtcaagga gaaggctaca ggggcactgt caataccatt 960
tggaatggaa ttccatgtca gcgttgggat tctcagtatc ctcacgagca tgacatgact 1020
cctgaaaatt tcaagtgcaa ggacctacga gaaaattact gccgaaatcc agatgggtct 1080
gaatcaccct ggtgttttac cactgatcca aacatccgag ttggctactg ctcccaaatt 1140
ccaaactgtg atatgtcaca tggacaagat tgttatcgtg ggaatggcaa aaattatatg 1200
ggcaacttat cccaaacaag atctggacta acatgttcaa tgtgggacaa gaacatggaa 1260
gacttacatc gtcatatctt ctgggaacca gatgcaagta agctgaatga gaattactgc 1320
cgaaatccag atgatgatgc tcatggaccc tggtgctaca cgggaaatcc actcattcct 1380
tgggattatt gccctatttc tcgttgtgaa ggtgatacca cacctacaat agtcaattta 1440
gaccatcccg taatatcttg tgccaaaacg aaacaattgc gagttgtaaa tgggattcca 1500
acacgaacaa acataggatg gatggttagt ttgagataca gaaataaaca tatctgcgga 1560
ggatcattga taaaggagag ttgggttctt actgcacgac agtgtttccc ttctcgagac 1620
ttgaaagatt atgaagcttg gcttggaatt catgatgtcc acggaagagg agatgagaaa 1680
tgcaaacagg ttctcaatgt ttcccagctg gtatatggcc ctgaaggatc agatctggtt 1740
ttaatgaagc ttgccaggcc tgctgtcctg gatgattttg ttagtacgat tgatttacct 1800
aattatggat gcacaattcc tgaaaagacc agttgcagtg tttatggctg gggctacact 1860
ggattgatca actatgatgg cctattacga gtggcacatc tctatataat gggaaatgag 1920
aaatgcagcc agcatcatcg agggaaggtg actctgaatg agtctgaaat atgtgctggg 1980
gctgaaaaga ttggatcagg accatgtgag ggggattatg gtggcccact tgtttgtgag 2040
caacataaaa tgagaatggt tcttggtgtc attgttcctg gtcgtggatg tgccattcca 2100
aatcgtcctg gtatttttgt ccgagtagca tattatgcaa aatggataca caaaattatt 2160
ttaacatata aggtaccaca gtcatag 2187

<210> 3

<211> 20

<212> DNA

<213> Artificial Sequence

<220>

<223> Description of Artificial Sequence: synthetic oligonucleotide

<400> 3

ccttgaaggg atttccctcc 20

## Description

### TECHNICAL FIELD

The present invention relates to a needleless syringe for Jet-injecting a pharmaceutical preparation containing genes and/or analogues thereof via a nozzle bore to thereby effect injection to a target site in a body. The present invention relates to a technique, in particular, in the field of gene therapy, for administering genes and/or analogues thereof such as HGF gene coding a hepatocyte growth factor or NF-κB decoy oligonucleotide into cells with high efficiency using this needleless syringe.

### BACKGROUND ART

A needleless syringe is a syringe that Jet-injects a predetermined amount of medical liquid via a nozzle bore provided at its tip end, thereby injecting the medical liquid to a target site in a patient body. Namely, injection using the needleless syringe is advantageous in that no pain is accompanied, a minimum injection scar is created, no needle stick accident because no injection needle is used for the injection, and the like.

Various forms of needleless syringe have been known. For example, JP-A 2003-507134 discloses a syringe for intradermally injecting a DNA based injecting substance. In this needleless syringe, the distance between the skin of patient and an orifice is kept in the range of 0.76 to 1.0 inches by means of an adaptor.

For example, JP-A 7-299140 discloses a disposable needleless syringe. In this needleless syringe, a reservoir having a cylindrical chamber for accommodating a medical liquid is integrally formed at a tip end of an elongated housing that forms a syringe main body.

JP-A 11-514242 (WO95/03844) and JP-A 2002-65851 disclose a needleless syringe. In this needleless syringe, a syringe main body includes a cartridge in which a medical liquid is accommodated in advance, and the cartridge includes a piston and an orifice. In order to strike the piston and initiate injection, an actuator is biased by a preliminarily compressed spring.

On the other hand, Japanese Patent No. 2577091 discloses a genetically obtained hepatocyte growth factor and a human HGF (hHGF) gene coding the same. This patent publication describes that hHGF, a hHGF-like substance or a fusion protein comprising hHGF can be obtained by expression of a template that is constructed by introducing a hHGF coding gene into an expression vector according to routine techniques, and the obtainable recombinant hHGF, a hHGF-like substance or a fusion protein comprising hHGF has the potential to become therapeutic agents for patients suffering from hepatic diseases, such as hepatic regeneration promoters, hepatic function improving agents, hepatitis therapeutic agents or hepatic cirrhosis inhibitors.

WO96/35430 discloses a therapeutic agent and a prophylactic agent containing NF-κB decoy oligonucleotide for diseases caused by NF-κB and examples of the diseases caused by NF-κB including ischemic diseases, inflammation diseases and autoimmune diseases.

### DISCLOSURE OF THE INVENTION

### Object of the Invention

There is a need for achieving higher therapeutic effect and no pain compared to injection using a conventional needle, by Jet-injecting a pharmaceutical preparation containing genes and/or analogues thereof via a nozzle bore to thereby effect injection to a target site in a body.

An object of the present invention is to provide a needleless syringe having, accommodated therein, a pharmaceutical preparation containing genes and/or analogues thereof.

An object of the present invention is to provide a safe needleless syringe capable of appropriately managing a liquid pharmaceutical preparation containing genes and/or analogues thereof and a syringe main body, while permitting any desired combination of kind of liquid pharmaceutical preparation to be injected and the syringe main body. More particularly, an object of the present invention is to provide a needleless syringe intended for one-time use.

Another object of the present invention is to provide a safe needleless syringe having, accommodated therein, a liquid pharmaceutical preparation containing genes and/or analogues thereof, wherein deformation of the syringe due to a compressed spring is prevented.

### Summary of the Invention

The present invention involves the following needleless syringes.
(1) A needleless syringe comprising:
   a medical agent chamber having one or multiple nozzle bore(s) and accommodated with a medical agent, and
   a syringe main body being provided with pressure application means to apply pressure on the medical agent toward a direction of the nozzle bore (s) and effect emission of the medical agent through the nozzle bore(s), wherein
   the medical agent is a pharmaceutical preparation containing genes and/or analogues thereof.

   Examples of the pressure application means include springs (various kinds of springs such as coil spring, leaf spring, dish spring, spring, and volute spring), compressors (utilizing air pressure), cylinders (various cylinders such as carbon dioxide gas cylinder), piezoelectric elements, explosives (chemical reaction, blasting = air pressure), electromagnetic power, oil pressure, water pressure and vapor, electric machineries (motors), generating machineries (engines), magnets (various types of magnets such as permanent magnet, and superconductive magnet), shape memory alloys, and the like.
(2) The needleless syringe according to (1), wherein the pharmaceutical preparation containing genes and/or analogues thereof is in a form of a carrier retaining the genes and/or analogues thereof.
(3) The needleless syringe according to (2), wherein the carrier is a metal particle.
(4) The needleless syringe according to (3), wherein the metal particle is a colloidal gold particle.
   The carrier is requested to be dense and biologically and chemically inert, and as such carrier, a gold particle is preferably used. Also particles of platinum, tungsten, iridium and the like may be used.
(5) A needleless syringe comprising:
   a medical liquid chamber having one or multiple nozzle bore(s), accommodated with a medical liquid, and provided with a piston so as to be located opposite to the nozzle bore (s) across the medical liquid; and
   a syringe main body being provided with a pressure application mechanism to apply pressure on the piston toward a direction of the nozzle bore (s) and effect emission of the medical liquid through the nozzle bore(s), wherein
   the medical liquid is a pharmaceutical preparation containing genes and/or analogues thereof.
(6) The needleless syringe according to (5), wherein the medical liquid is a pharmaceutical preparation containing a gene coding an angioproliferator or an angiogenesis protein.
(7) The needleless syringe according to (6), wherein the angioproliferator or the angiogenesis protein is one selected from the group consisting of scatter factor/hepatocyte growth factor (HGF), vascular endothelial growth factor (VEGF), fibroblast growth factor (FGF), transforming growth factor (TGF), erythropoietin (EPO), angiogenin, pleiotrophin (PTN, HB-GAM), midkine, placental growth factor protein (PIGF), platelet-derived growth factor (PDGF), Del-1 (Developmentally Regulated Endothelial Cell Locus-1), angiopoietin, folistatin, granulocyte colony stimulating factor (G-CSF), leptin, insulin-like growth factor (IGF), chicken ovalbumin upstream promoter-transcription factor II (COUP-TFII), endothelial NO synthetase (eNOS), inductive NO synthetase (iNOS), human monocyte chemotactic factor (monocyte chemotactic protein-1, MCP-1), proliferin and ephrin.
(8) The needleless syringe according to (5), wherein the medical liquid is a pharmaceutical preparation containing a gene coding HGF.
(9) The needleless syringe according to (8), wherein the pharmaceutical preparation containing a gene coding HGF is a pharmaceutical preparation containing an expression vector into which a gene coding HGF is inserted.
(10) The needleless syringe according to (9), wherein the expression vector is one selected from the group consisting of plasmids, adenovirus vectors and HVJ-E vectors (Hemagglutinating Virus of Japan-envelope vectors). HVJ is also called Sendai Virus.
(11) The needleless syringe according to (5), wherein the medical liquid is a pharmaceutical preparation containing a biologically active oligonucleotide.
(12) The needleless syringe according to (11), wherein the biologically active oligonucleotide is decoy, antisense oligonucleotide, aptamer or RNAi effector.
(13) The needleless syringe according to (12), wherein the antisense oligonucleotide is one selected from the group consisting of ISIS-2302, ISIS-2503, ISIS-2922, ISIS-3521, ISIS-5132, ISIS-14803, ISIS-14838, ISIS-15839, G-3139, IN-3001, GPI-2A and EPI-2010.
(14) The needleless syringe according to (12), wherein the decoy is a transcription factor inhibiting decoy oligonucleotide.
(15) The needleless syringe according to (14), wherein the transcription factor is one selected from the group consisting of NF-κB, E2F, AP-1, STAT-1, STAT-6, GATA-3 and Ets.
(16) The needleless syringe according to (5), wherein the medical liquid is a pharmaceutical preparation containing NF-κB decoy oligonucleotide.
   In the above needleless syringes of the present invention, it is possible to Jet-inject a pharmaceutical preparation containing genes and/or analogues thereof as a medical liquid via the nozzle bore(s) to thereby effect injection to a target site in a body.
(17) The needleless syringe according to any one of (5) to (16), wherein the medical liquid chamber is formed inside a nozzle ampoule that is detachably attached to the syringe main body.
   In the needleless syringe of this invention, since the nozzle ampoule and the syringe main body are detachable from each other, when the syringe is stocked and stored, it is possible to store the nozzle ampoule and the syringe main body separately while the nozzle ampoule filled with the medical liquid is detached from the syringe main body. Accordingly, the nozzle ampoule can be refrigerated at an optimum temperature for the medical liquid filling the same, and the syringe main body can be stored at normal temperature, so that appropriate management can be easily conducted. In use, a nozzle ampoule filled with a requested medical liquid is chosen, and then attached to the syringe main body. Accordingly, the syringe main body can be in combination with any desired kinds of medical liquid. Furthermore, since a predetermined amount of medical liquid is charged in advance in the nozzle ampoule, transferring of the medical liquid before use is no longer required and hence accurate dose amount is ensured in hygienic manner.
(18) The needleless syringe according to (17), wherein
   the nozzle ampoule has an ampoule opening in which the piston is exposed, the opening verge of the ampoule being formed with a plurality of convex fitting portions,
   the syringe main body has a hollow housing accommodating the pressure application mechanism, the housing having a housing opening communicated with the ampoule opening, the opening verge of the housing being formed with concave fitting portions corresponding to the convex fitting portions provided in the nozzle ampoule, and
   the convex fitting portions and the concave fitting portions are fitted or removed by rotating the nozzle ampoule and the housing in mutually opposite directions.
   In the needleless syringe of this invention, by such a simple operation as rotating at least one of the nozzle ampoule and the housing, it is possible to detach the nozzle ampoule from the syringe main body.
(19) The needleless syringe according to (17) or (18), wherein the pressure application mechanism is provided with, a pressure application member which is movable in the direction of approaching/leaving the piston, a compression spring that biases the pressure application member toward the direction of approaching the piston, and a holding member that releasably holds movement of the pressure application member by the spring in the state that the spring is compressed.
   In the needleless syringe according to this invention, release of spring force by the holding member causes the medical liquid to be Jet-injected through the nozzle bore (s).
(20) The needleless syringe according to (19), wherein
   in the housing formed are a spring chamber having a housing opening at one end of the spring chamber, and a releasing chamber partitioned by a partition wall having a through hole from the spring chamber,
   the pressure application member is provided with a spring receiver located in the spring chamber and abutted against the piston, and a rod extending from the spring receiver through the through hole of the partition wall and projecting into the release chamber, an engaging projection being formed on an outer surface of projecting end of the rod in such a dimension that the engaging projection can not pass through the through hole of the partition wall, the spring being a coil spring interposed between the partition wall and the spring receiver so as to surround the rod, and
   the holding member has a stopper face against which the engaging projection of the rod is abutted in the state that the spring is compressed.
   In the needleless syringe of this invention, the engaging projection of the rod is abutted against the stopper face of the holding member, whereby the spring can be held in a compressed state. In addition, since the spring receiver of the pressure application member is abutted against the piston in the state that the spring is compressed, when the holding by the holding member is released, pressure can be applied on the piston without generation of impact by the pressure application member. On the condition that a gap generates between the piston and the pressure application member, when the pressure application member comes into collision with the piston, an impact occurs, resulting that a loud discharge sound is made or a great impact is transmitted to the hand. Also there is a risk that the piston is damaged. Furthermore, since the engaging projection of the rod is formed in such a dimension that it cannot pass through the through hole of the partition wall, when holding by the holding member is released, the rod will stop at a predetermined position. In this manner, administration of an accurate amount of medical liquid and safety after administration of medical liquid are secured. The moving distance of the rod may be appropriately selected depending on the dose amount of medical liquid, namely, the moving distance of the piston.
(21) The needleless syringe according to (20), wherein the holding member is provided with a ring portion received by the partition wall in the releasing chamber so as to surround the rod, and a cylindrical part provided upright on the ring portion and having a tip end face designed as the stopper face, and a plurality of slits are formed so as to longitudinally pass through the cylindrical part.
   The cylindrical part may be formed of, for example, an elastic material such as hard resin. In the needleless syringe of this invention, holding by the holding member can be released by deformation of the part sandwiched between adjacent slits of the cylindrical part.
(22) The needleless syringe according to (21), wherein opposite faces of the each slit are partially connected via a breakable portion.
   In the needleless syringe of this invention, since opposite faces of the each slit are partially connected via a breakable portion, holding by the holding member can be more securely achieved while overcoming the force of the compressed spring. Once the breakable portion breaks during injection of the medical liquid, the holding member is no longer used again. Accordingly, the syringe is disposable, and accident such as infection caused by reuse of the syringe can be prevented.
(23) The needleless syringe according to (21) or (22), wherein the housing has an end wall that opposes the partition wall while leaving a space therebetween, a releasing member is held so as to be able to approach/leave the stopper face through a holding hole formed in the end wall, the stopper face is formed into a tapered recess that spreads as the stopper face comes closer to the releasing member, and the releasing member is provided with a push-spreading face that is formed into a tapered projection corresponding to the stopper face.
   In the needleless syringe of this invention, since the holding of the spring force of the holding member can be released only by such a simple operation as pushing down the releasing member, injection itself can be easily conducted.
(24) The needleless syringe according to (20), wherein the holding member comprises a stopper piece that is movably inserted in a locking hole and has the stopper face in an inward end, the locking hole penetrating a peripheral wall of the releasing chamber from inside to outside thereof and extending in the direction orthogonal to the rod.
   In the needleless syringe of this invention, it is possible to release the holding of the spring force of the holding member by displacing the stopper piece. Therefore, injection itself can be conducted in a simple manner.
(25) The needleless syringe according to (24), wherein on an outer surface of the peripheral wall of the releasing chamber is formed a guide groove which extends in the direction orthogonal to the locking hole and permits an outward end of the locking hole to open at a bottom face of the guide groove, a locking member is movably fitted into the guide groove, in an inner lateral surface of the locking member is formed a recess that allows insertion of an outward end of the stopper piece.
   In the needleless syringe of this invention, the stopper piece can be automatically moved toward the releasing position by action of the spring force by displacing the locking member.
(26) The needleless syringe according to any one of (20) to (25), wherein a safety cap which is attached to the syringe main body is provided in place of the nozzle ampoule, the safety cap having a convex fitting portion which is equivalent to the convex fitting portion of the nozzle ampoule, and a gap is kept between the engaging projection of the rod and the stopper face of the holding member in the state that the convex fitting portion of the safety cap is fitted into the concave fitting portion of the housing.
   In the needleless syringe of this invention, the safety cap is attached to the syringe main body during storage of the syringe and until the nozzle ampoule is attached to the syringe main body just before use, and thereby the gap between the engaging projection of the rod and the stopper face of the holding member is kept. In other words, the holding member does not need to always have a strong spring force, but may have the strong spring force in the case of use. Therefore, even when they are placed in environments of high temperature and high humidity, undesired deformation can be prevented, so that safety and accuracy of the syringe is ensured.
(27) The needleless syringe according to any one of (5) to (16), wherein the medical liquid chamber is integrated with the syringe main body. The medical liquid chamber may be formed within the syringe main body, or the medical liquid chamber may be formed inside the nozzle ampoule that is integrated with the syringe main body, as described below.
(28) The needleless syringe according to (27), wherein the medical liquid chamber is formed inside the nozzle ampoule that is integrated with the syringe main body.
(29) The needleless syringe according to (27) or (28), wherein
   the syringe main body has a hollow housing accommodating the pressure application mechanism,
   the pressure application mechanism is provided with a pressure application member which is movable in the direction of approaching/leaving the piston, a compression spring that biases the pressure application member toward the direction of approaching the piston, and a holding member that releasably holds movement of the pressure application member by the spring in the state that the spring is compressed,
   in the housing are formed a spring chamber having a housing opening at one end of the spring chamber, and a releasing chamber that is partitioned by a partition wall having a through hole from the spring chamber,
   the pressure application member is provided with a spring receiver located in the spring chamber and abutted against the piston, and a rod extending from the spring receiver through the through hole of the partition wall and projecting into the release chamber, an engaging projection being formed on an outer surface of projecting end of the rod in such a dimension that the engaging projection can not pass through the through hole of the partition wall, the spring being a coil spring interposed between the partition wall and the spring receiver so as to surround the rod, and
   the holding member is provided with a ring portion received by the partition wall so as to surround the rod in the releasing chamber, and a cylindrical part provided upright on the ring portion, a tip end face of the cylindrical part being designed as a stopper face against which the engaging projection of the rod is abutted in the state that the spring is compressed, a plurality of slits being formed so as to longitudinally pass through the cylindrical part.
   In the needleless syringe of this invention, similar effects are realized as is the cases of needleless syringes according to (15), (16) and (17) except for the effects based on the fact that the nozzle ampoule and the syringe main body are detachable.
(30) The needleless syringe according to (29), wherein opposite faces of the each slit are partially connected.
   In the needleless syringe of this invention, similar effects are realized as is the case of needleless syringe according to (18) except for the effects based on the fact that the nozzle ampoule and the syringe main body are detachable.
(31) The needleless syringe according to (29) or (30), wherein the housing has an end wall that opposes the partition wall while leaving a space therebetween, a releasing member is held so as to be able to approach/leave the stopper face through a holding hole formed in the end wall, the stopper face is formed into a tapered recess that spreads as the stopper face comes closer to the releasing member, and the releasing member is provided with a push-spreading face that is formed into a tapered projection corresponding to the stopper face.
   In the needleless syringe of this invention, similar effects are realized as is the case of needleless syringe according to (19) except for the effects based on the fact that the nozzle ampoule and the syringe main body are detachable.
(32) The needleless syringe according to (27) or (28), wherein
   the syringe main body has a hollow housing accommodating the pressure application mechanism,
   the pressure application mechanism is provided with a pressure application member which is movable in the direction of approaching/leaving the piston, a compression spring that biases the pressure application member toward the direction of approaching the piston, and a holding member that releasably holds movement of the pressure application member by the spring in the state that the spring is compressed,
   in the housing are formed a spring chamber having a housing opening at one end of the spring chamber, and a releasing chamber that is partitioned by a partition wall having a through hole from the spring chamber,
   the pressure application member is provided with a spring receiver located in the spring chamber and abutted against the piston, and a rod extending from the spring receiver through the through hole of the partition wall and projecting into the release chamber, an engaging projection being formed on an outer surface of projecting end of the rod in such a dimension that the engaging projection can not pass through the through hole of the partition wall, the spring being a coil spring interposed between the partition wall and the spring receiver so as to surround the rod, and
   the holding member comprises a stopper piece that is movably inserted in a locking hole, the locking hole penetrating a peripheral wall of the releasing chamber from inside to outside thereof and extending in the direction orthogonal to the rod, a stopper face against which the engaging projection of the rod is abutted in the state that the spring is compressed being formed in an inward end of the stopper piece.
   In the needleless syringe of this invention, similar effects are realized as is the cases of needleless syringes according to (19), (20) and (21) except for the effects based on the fact that the nozzle ampoule and the syringe main body are detachable.
(33) The needleless syringe according to (32), wherein on an outer surface of the peripheral wall of the releasing chamber is formed a guide groove which extends in the direction orthogonal to the locking hole and permits an outward end of the locking hole to open at a bottom face of the guide groove, a locking member is movably fitted into the guide groove, in an inner lateral surface of the locking member is formed a recess that allows insertion of an outward end of the stopper piece.

In the needleless syringe of this invention, similar effects are realized as is the case of needleless syringe according to (25) except for the effects based on the fact that the nozzle ampoule and the syringe main body are detachable.

The present invention further involves the following aspects of the invention.

A therapeutic and prophylactic method for diseases, comprising the step of administering an effective amount of liquid pharmaceutical preparation containing genes and/or analogues thereof to a mammal using any of the above needleless syringe.

A therapeutic, improving and prophylactic method for liver diseases or disease to which angiogenesis is effective, comprising the step of administering an effective amount of liquid pharmaceutical preparation containing a gene coding HGF to a mammal using any of the above needleless syringe.

A therapeutic and prophylactic method for diseases caused by NF-κB, comprising the step of administering an effective amount of liquid pharmaceutical preparation containing NF-κB decoy oligonucleotide to a mammal using any of the above needleless syringe.

By using the needleless syringe of the present invention, it is possible to transfer genes and/or analogues thereof such as HGF gene coding hepatocyte growth factor and NF-κB decoy oligonucleotide into cells with high efficiency. Moreover, patients feel no pain, and injection scar is very small.

According to a preferred embodiment of the present invention, a safe needless syringe is provided wherein the medical liquid and the syringe main body can be properly managed separately, and any desired combination of kind of medical liquid and the syringe main body is permitted.

Since a preferred embodiment of the needleless syringe in the present invention has such a mechanism that strong spring force will never act on the holding member during storage of the syringe main body, high safety during storage is ensured. Furthermore, since the pressure application member has a pressure application mechanism which stops at a predetermined position after injection of the medical liquid, high safety after emission of the medical liquid is ensured. Furthermore, since the holding member cannot be reused after emission of the medical liquid, an accident such as infection due to reuse of the syringe can be prevented by making the syringe disposable.

In a needleless syringe of the type that the nozzle ampoule and the syringe main body are not detachable from each other, similar effects described above can be realized except for the effects based on the fact that the nozzle ampoule and the syringe main body are detachable.

Since spring force generally correlates with injection power of medical liquid, by appropriately adjusting the spring force, it is possible to obtain a needleless syringe which is optimum for the object and site of therapy. In addition, by appropriately changing the diameter, length and the like shape of the nozzle bore depending on the kind of the medical liquid, a needleless syringe which is optimum for the medical liquid is obtained. The needleless syringe of the present invention may readily be applied as well as normal application for injection together with rigid endoscopes such as arthroscope, laparoscope and thoracoscope, or applied during surgery, or applied in catheter treatment, and may be used in direct contact with a site to be treated in a body. In this manner, it is possible to directly administer (Cellular transfection) the medical liquid to the site to be treated in the body.

### BREIF DESCRIPTION OF THE DRAWINGS

Fig. 1 is a longitudinal sectional view of one example of a syringe of the present invention in use.
Fig. 2 is a longitudinal sectional view of the same syringe in storage.
Fig. 3 is a traverse sectional view taken along line III-III in Fig. 1.
Fig. 4 is a traverse sectional view taken along line IV-IV in Fig. 1.
Fig. 5 is an enlarged perspective view of a holding member of the same syringe.
Fig. 6 is a longitudinal sectional view of a holding member in a modified example of the syringe of the present invention.
Fig. 7 is a side view of the part shown in Fig. 6.
Figs. 8 is micrograph of epidermal layer (imaging magnification: (a) x40, (b) x200) showing result of Cellular transfection of Venus gene to epidermal tissue using the syringe of the present invention.
Figs. 9 is micrograph of epidermal layer (imaging magnification: (a) x40, (b) x200) showing result of Cellular transfection of LacZ gene to epidermal tissue using the syringe of the present invention.
Fig. 10 is a view showing the results of Cellular transfection of HGF gene using the needleless syringe of the present invention and a conventional syringe with a needle.

### MODES FOR CARRYING OUT THE INVENTION

In the present invention, various forms of needleless syringe may be used. For example, as a pressure application mechanism that applies pressure on the piston toward the nozzle bore to effect emission of the medical liquid through the nozzle bore, it may employ any systems including compression or drawing spring system, gas pressure (carbon dioxide gas, nitrogen oxide gas) system, explosive system, electromagnetic system, piezoelectric system and the like. Those employing a gas pressure system are found in JP-A 61 265147, JP-A 3-503374 (WO89/08469), JP-A 8-509604 (WO94/24263) and the like. Those employing a compression spring system are found in JP-A 2000-126293, JP-A 2001-187139, JP-A 2001-61964 and the like. Among these, those employing the compression spring system which is safe and easy to handle are preferred.

In the present invention, the needleless syringe may be used as either those capable of one-time use or repeated use. From the viewpoint of preventing an accident such as infection by the reuse of the syringe, those capable of one-time use are preferred.

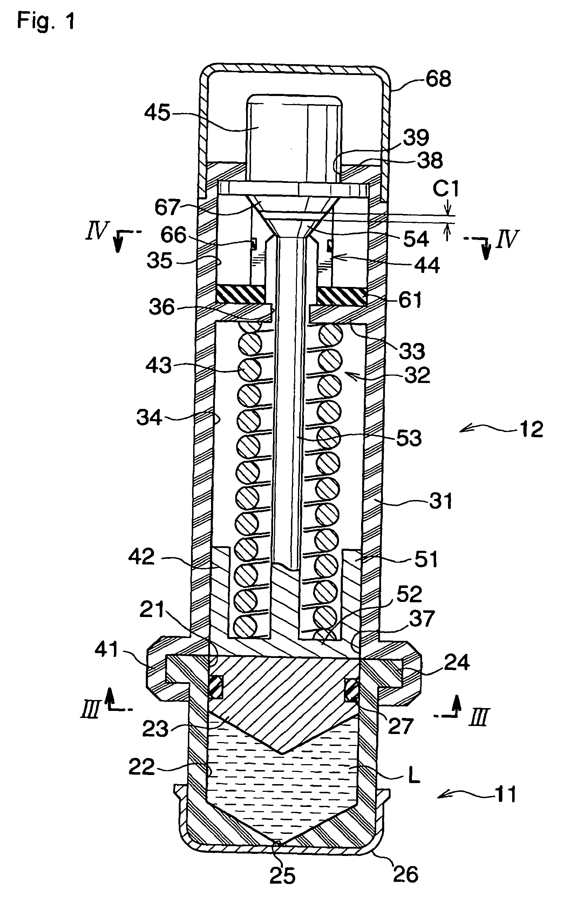

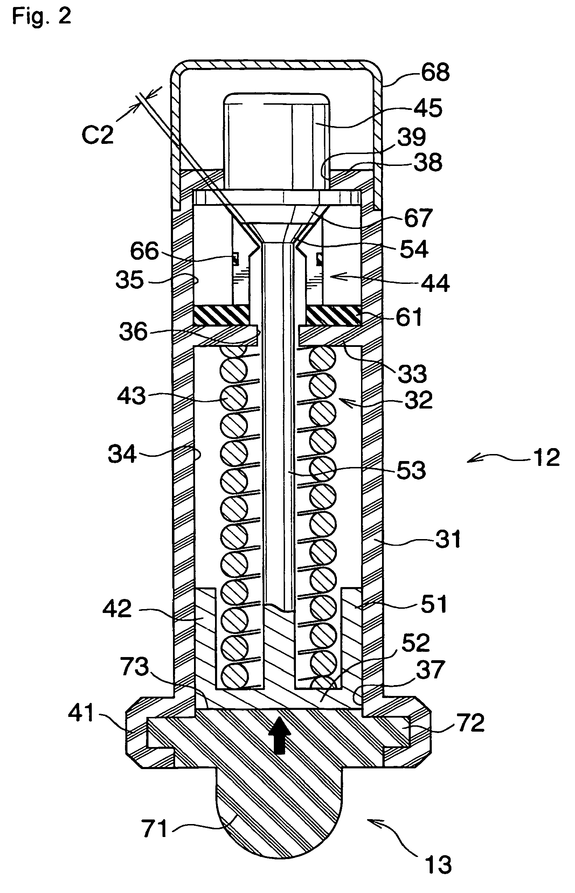

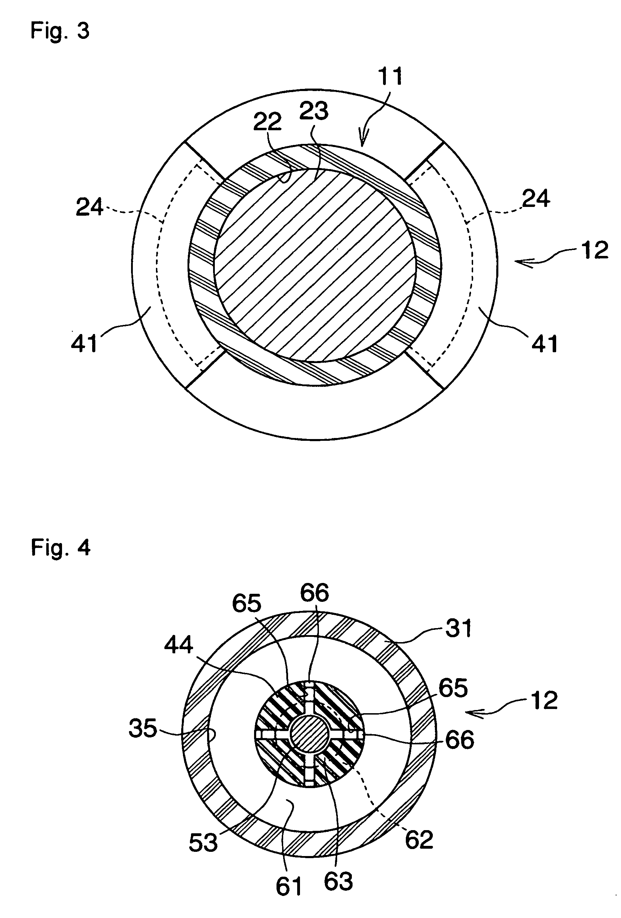

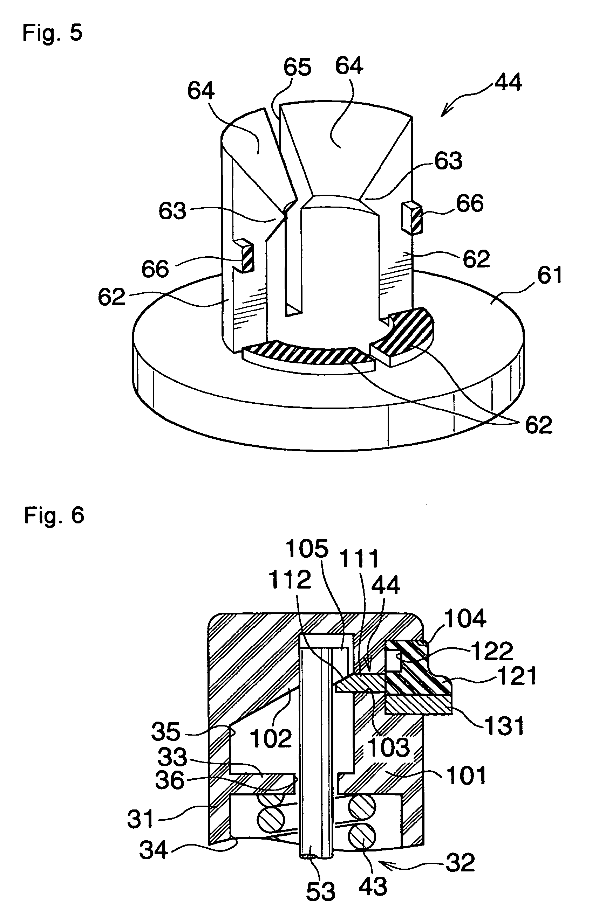

Preferred embodiments of the present invention will now be described with reference to the attached drawings. Fig. 1 is a longitudinal sectional view of one example of the syringe of the present invention in use. Fig. 2 is a longitudinal sectional view of the same syringe in storage. Fig. 3 is a traverse sectional view taken along line III-III in Fig. 1. Fig. 4 is a traverse sectional view taken along line IV-IV in Fig. 1. Fig. 5 is an enlarged perspective view of a holding member of the same syringe. In the following description, the up-and-down direction is based on Fig. 1.

Referring to Fig. 1, a needleless syringe includes a nozzle ampoule 11, and a syringe main body 12 to which the nozzle ampoule 11 is attached. Referring to Fig. 2, the syringe main body 12 has a safety cap 13 attached, in place of the nozzle ampoule 11, to the syringe main body 12.

The nozzle ampoule 11 comprises a cylinder having a bottom and having an ampoule opening 21 at its upper end, and the interior of the cylinder serves as a medical liquid chamber 22. A bottom surface of the medical liquid chamber 22 of the nozzle ampoule 11 is formed into an upwardly concave circular conical surface. The medical liquid chamber 22 accommodates a medical liquid L and a piston 23 thereon. The nozzle ampoule 11 is preferably formed of a transparent material which is compatible to the medical liquid L, e.g., transparent hard plastic such as polycarbonate or glass in order that the medical liquid L may be visually checked.

The medical liquid L is a liquid pharmaceutical preparation containing genes and/or analogues thereof. Specific description for the medical liquid L will be given later.

In an edge part of the ampoule opening 21, two convex fitting portions 24 in shape of partial ring are provided so as to project to mutually opposite lateral sides. A sandwiching angle of each convex fitting portion 24 is a little under 90 degrees (see Fig. 3). In a center part of the lower face of the nozzle ampoule 11, a nozzle bore 25 is formed so as to communicate with the medical liquid chamber 22. In the lower part of the nozzle ampoule 11, a nozzle protecting cap 26 is provided. The protecting cap 26 is usually formed of plastic, but it is preferably attached with a thin rubber material e.g., silicone rubber for sealing, which is not shown, in a center part where it contacts the nozzle bore 25. The rubber material can prevent the medical liquid from leaking.

In the depicted example, one nozzle bore 25 is formed, however, multiple nozzles may be formed as described in JP-A 2000-126293.

The piston 23 is usually formed of plastic, and is a horizontal disc that is fitted in the peripheral wall of the nozzle ampoule 11 so as to be able to slide in the up-and-down direction. The lower face of the piston 23 is formed into a downwardly projecting circular conical face which coincides with the bottom face of the medical liquid chamber 22. The piston 23 is attached with an O ring 27 on its outer peripheral surface. The top face of the piston 23 is flush with the upper end face of the nozzle ampoule 11.

The syringe main body 12 is provided with an upright cylindrical housing 31, and a pressure application mechanism 32 which is accommodated in the housing 31 and applies pressure on the piston 23 downwardly. The housing 31 is preferably made of metal in order to keep the strength, however, it may be made of plastic.

The interior of the housing 31 is partitioned into a lower spring chamber 34 and an upper releasing chamber 35 by a horizontal partition wall 33. In a center part of the partition wall 33, a through hole 36 is formed. In a lower end of the spring chamber 34, a housing opening 37 which coincides with the ampoule opening 21 is formed. In an upper end of the releasing chamber 35, an end wall 38 is provided. In a center part of the end wall 38, a holding hole 39 is formed.

In an edge part of the housing opening 37, two concave fitting portions 41 into which the respective convex fitting portions 24 are fitted are formed. A sandwiching angle of each concave fitting portion 41 is almost equal to that of the convex fitting portion 24.

The pressure application mechanism 32 includes a pressure application member 42 held in the housing 31 so as to be able to move in the up-and-down direction, a compression coil spring 43 biasing the pressure application member 42 downwardly, a holding member 44 that releasably holds the movement of the pressure application member 42 by the spring 43 in the state that the spring 43 is compressed, and a releasing member 45 that releases the holding by the holding member 44.

The pressure application member 42 includes:
a horizontal disc-like spring receiver 52, that is made to abut against the top face of the piston 23 and has at its outer periphery a rising guide cylinder 51 which is made to contact slidingly with the inner peripheral surface of the spring chamber 34; and
a vertical rod 53 that is provided upright in a center part of the spring receiver 52 and has an upper end part projecting into the releasing chamber 35 through the through hole 36. In an upper end part of the rod 53, a circular outward engaging projection 54 is provided. The engaging projection 54 is dimensioned such that it cannot pass through the through hole 36 of the partition wall 33. The lower lateral face of the projection 54 is shaped into an upwardly spread taper. The pressure application member 42 is preferably made of metal.

The spring 43 is accommodated in a compressed state in the spring chamber 34 such that it surrounds the rod 53 and is interposed between the partition wall 33 and the spring receiver 52.

The holding member 44 is integrally formed, for example, of hard plastic, and includes a ring portion 61 that surrounds the rod 53 and received by the partition wall 33, and a vertical cylindrical part 62 provided upright on the top face of the ring portion 61 so as to be concentric with the rod 53, as is specifically shown in Figs. 4 and 5. In an upper end part of the inner surface of the cylindrical part 62, a circular inward engaging projection 63 is provided. An upper lateral face of the inward engaging projection 63 is formed into a concave stopper face 64 of an upwardly spread taper. An inner edge part of the stopper face 64 engages with the lower lateral face of the outward engaging projection 54 of the rod 53 from lower side. At every quarter point in the circumferential direction of the cylindrical part 62, slits 65 are formed so as to traverse the inward engaging projection 63 as well. The opposite faces of each slit 65 are partially connected via an breakable portion 66 which can easily break at about middle level along the height. That is, the cylindrical part 62 consists of four pieces which are partially connected to each other via the breakable portion 66. As the plastic material for the holding member 44, an elastic material is selected. In the depicted example, the cylindrical part 62 is divided into four equal parts by the slits 65 in a circumferential direction. However, the cylindrical part 62 is not necessarily divided equally insofar as it is divided into plural pieces.

The releasing member 45 is made from a perpendicular plastic cylinder having closed ends, fitted in the holding hole 39 so as to be able to move in the up-and-down direction. In a part closer to the outer periphery of bottom face of the releasing member 45, a downwardly reducing tapered convex push-spreading face 67 which is designed to coincide with the stopper face 64 is formed. On an upper end of the housing 31, a protecting cap 68 is attached so as to surround the releasing member 45.

In the state that the push-spreading face 67 is made to abut against the stopper face 64, there is a small breakage gap C1 between the upper end face of the rod 53 and the center part of the bottom face of the releasing member 45.

The safety cap 13 is made from a plastic disc member having a hemispherical gripping portion 71 integrally formed in a center part of the lower face. On an outer periphery of the safety cap 13, two convex fitting portions 72 having the same shape with the convex fitting portion 24 of the nozzle ampoule 11 are equally formed. In a center part of the top face of the safety cap 13 is formed a circular projecting push-up portion 73 closely fitted into the ampoule opening 21 and having a height corresponding to the breakage gap C1. The convex fitting portions 72 of safety cap 13 is brought into fitted into the concave fitting portion 41 of the housing 31, whereby attachment of the safety cap 13 to the housing 31 is accomplished. In this state, when compared to the state that the nozzle ampoule 11 is attached to the housing 31, the pressure application member 42 is pushed up by a level corresponding to the height of the push-up portion 73. Accordingly, a safety gap C2 in place of the breakage gap C1 is formed between the outward engaging projection 54 of the rod 53 and the inward engaging projection 63 of the holding member 44. When the safety gap C2 is formed, the spring force of the spring 43 does not act on the holding member 44 at all. Therefore, greater safety is secured during storage of the syringe.

When the syringe is not used, namely, in the period from production of syringe to directly before using the syringe in usual, the safety cap 13 is attached to the syringe main body 12. The nozzle ampoule 11 having, accommodated therein, a medical liquid may be separately stored in a refrigerator or the like.

In use of the syringe, the housing 31 and the safety cap 13 are rotated in mutually opposite directions by about 90 degrees. This makes the convex fitting portion 72 of the safety cap 13 leave the concave fitting portion 41 of the housing 31, and the safety cap 13 is removed from the housing 31. As a result, the breakage gap C1 is formed in place of the safety gap C2. In this state, the lower face of the spring receiver 52 is flush with the lower end face of the housing 31.

Next, in place of the safety cap 13, the nozzle ampoule 11 having, accommodated therein, a medical liquid is attached to the housing 31. Locating the two convex fitting portions 24 of the nozzle ampoule 11 between the two concave fitting portions 41 of the housing 31, the housing 31 and the safety cap 13 are rotated in mutually opposite directions by about 90 degrees, and then both of the fitting portions 24 and the fitting portions 41 come into fitted with each other.

After removing the upper and lower protecting caps 26 and 68, the nozzle bore 25 is pushed against a skin or the like to which injection is conducted, and the releasing member 45 is pushed down. When the releasing member 45 is pushed down, the push-spreading face 67 pushes the stopper face 64 diagonally and downwardly so as to spread the cylindrical part 62 upwardly. As a result, first, all or part of the breakable portions 66 are broken. Subsequently, as the releasing member 45 is pushed down, the cylindrical part 62 is further spread upwardly, and each piece of the cylindrical part 62 elastically deforms and outwardly falls. In accordance with this, the stopper face 64 gradually moves outward, and finally the inward engaging projection 63 of the stopper face 64 and the outward engaging projection 54 of the rod 53 are disengaged. As a result, the pressure application member 42 is swiftly pushed down by the force of the spring 43, and simultaneously, the piston 23 is pushed down, whereby the medical liquid L is compressed under high pressure and emitted at high speed via the nozzle bore 25 to thereby effect subcutaneous injection. Since the engaging projection 54 of the rod 53 cannot pass through the through hole 36 of the partition wall 33, the pressure application member 42 is stopped at a predetermined position after emission of the medical liquid, which secures the safety.

In the above, the description was given for the needleless syringe in which the nozzle ampoule and the syringe main body are detachable from each other. However, other arrangements except for the arrangement that the nozzle ampoule and the syringe main body are detachable from each other, especially arrangements of the pressure application mechanism 32, in particular, the holding member 44, the breakable portion 66, the pressure application member 42 and the releasing member 45 are applicable to needleless syringes in which a nozzle ampoule is fixed to a syringe main body.

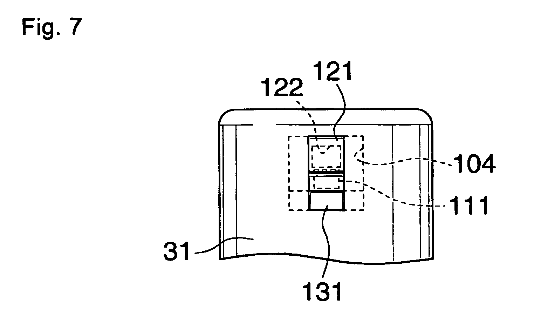

Figs. 6 and 7 show a modified example of the holding member 44. In this modified example, a thick holder 101 is provided in a part of a peripheral wall of the releasing chamber 35 of the housing 31, and a guiding portion 102 is provided opposite to the holder 101 while interposing the rod 53 therebetween. The guiding portion 102 is made to slidingly contact the rod 53.

The holder 101 is formed with a locking hole 103 extending in a direction orthogonal to the rod 53 so as to penetrate the holder 101 from inside to outside. On the other hand, in an outer surface of the peripheral wall of the releasing chamber 35, an interior enlarging guide groove 104 is formed vertically. At the bottom face of the guide groove 104, an outward end of the locking hole 103 opens. In an outer surface of an upper end part of the rod 53, an engaging protrusion 105 extending toward the holder 101 is provided. The engaging protrusion 105 has an inclined lower face. The engaging protrusion 105 has such a dimension that cannot pass through the through hole 36 of the partition wall 33.

The holding member 44 is formed of a stopper piece 111 that is movably inserted in the locking hole 103 and has an upward stopper face 112 in its inward end.

In the guide groove 104, a locking member 121 is fitted so as to be able to slide in the up-and-down direction. In an inner lateral face of the locking member 121, a recess 122 that allows insertion of the outward end of the stopper piece 111 is formed.

In the state that the locking member 121 is made to abut against the upper end face of the guide groove 104, the recess 122 is blocked by the bottom face of the guide groove 104. Further, in this state, there is a gap between the lower end face of the guide groove 104 and the locking member 121, and a safety pin 131 is removably inserted into this gap.

This safety pin 131 restricts downward movement of the locking member 121, and at the same time, the locking member 121 restricts outward movement of the stopper piece 111. The safety pin 131 is removed, and then free movement of the locking member 121 is allowed. The locking member 121 is displaced downward so as to make the heights of the stopper piece 111 and the recess 122 coincide with each other, and then outward movement of the stopper piece 111 is allowed. Since the rod 53 is downwardly biased by the force of the spring 43, the stopper piece 111 is pushed outward by the engaging protrusion 105 of the rod 53. As a result, engagement between the engaging protrusion 105 and the stopper piece 111 is cancelled, and the rod 53 is swiftly pushed down by the force of the spring 43, and simultaneously the piston 23 is pushed down. As a result, the medical liquid L is compressed under high pressure, and emitted at high speed via the nozzle bore 25 to accomplish the subcutaneous injection. Since the engaging protrusion 105 of the rod 53 cannot pass through the through hole 36 of the partition wall 33, the pressure application member 42 is stopped at a predetermined position after emission of the medical liquid to ensure the safety.

The modified example of the holding member 44 shown in Figs. 6 and 7 is applied to both a needleless syringe in which a nozzle ampoule and a syringe main body are detachable and a needleless syringe in which a nozzle ampoule is fixed to a syringe main body.

In the case of a needleless syringe in which a nozzle ampoule and a syringe main body are detachable from each other, the modified example of Fig. 6 represents the state during use of the syringe, and corresponds to Fig. 1. For storing the syringe shown in the modified example of Fig. 6, the safety cap 13 is attached to the syringe main body 12 as is shown in Fig. 2. By attaching the safety cap 13, the rod 53 is pushed up by a level corresponding to the height of the push-up portion 73 the cap 13, and a safety gap (corresponding to the safety gap C2 in Fig. 2) is formed between the engaging protrusion 105 of the rod 53 and the stopper piece 111. Since the spring force of the spring 43 does not act on the stopper piece 111 at all because of the safety gap, greater safety is secured during storage of the syringe.

A modified example of the holding member is presented in the foregoing description, however, the needleless syringe of the present invention is not limited to this, and may be embodied in various forms. Therefore, the above embodiments are merely illustrative, and should not be interpreted in a limitative way.

Next, description will be given for the medical liquid L accommodated in the medical liquid chamber. The medical liquid L is a pharmaceutical preparation liquid containing genes and/or analogues thereof.

The pharmaceutical preparation containing genes and/or analogues thereof is not particularly limited, however, preferably there may be mentioned a pharmaceutical preparation containing a gene coding an angioproliferator or an angiogenesis protein. More specific examples of the angioproliferator or angiogenesis protein include scatter factor/hepatocyte growth factor (HGF), vascular endothelial growth factor (VEGF), fibroblast growth factor (FGF), transforming growth factor (TGF), erythropoietin (EPO), angiogenin, pleiotrophin (PTN, HB-GAM), midkine, placental growth factor protein (PIGF), platelet-derived growth factor (PDGF), Del-1 (Developmentally Regulated Endothelial Cell Locus-1), angiopoietin, folistatin, granulocyte colony stimulating factor (G-CSF), leptin, insulin-like growth factor (IGF), chicken ovalbumin upstream promoter-transcription factor II (COUP-TFII), endothelial NO synthetase (eNOS), inductive NO synthetase (iNOS), human monocyte chemotactic factor (monocyte chemotactic protein-1, MCP-1), proliferin, ephrin, and the like. Among these, HGF is more preferable.

Preferred examples of the medical liquid include pharmaceutical preparations containing a gene coding HGF as a main component. The gene coding HGF is a gene that codes hepatocyte growth factor represented by an amino acid sequence shown in SEQ ID NO. 1 of the sequence list. Also the gene coding HGF is a gene that codes hepatocyte growth factor represented by a base sequence shown in SEQ ID NO. 2 of the sequence list.

The gene coding HGF may be introduced into an expression vector by routine techniques. Examples of the expression vector include plasmids, adenovirus vectors, HVJ-E vectors and the like.

A pharmaceutical preparation containing a gene coding HGF as a main component has angiogenetic function owing to growth of vascular endothelial cell, and is effective in prophylaxis and therapy of the artery diseases such as arteriosclerosis and peripheral circulatory failure, myocardial infarction, peripheral vascular obstruction and the like. Furthermore, such pharmaceutical preparation containing a HGF coding gene as a main component also has a function of promoting repair of articular cartilage cell, and is effective in prophylaxis and therapy of osteodystrophy, osteoarthritis, sport injury and the like.

In the present invention, a gene coding HGF having partial deletion, change, addition and the like modification made unless the above effects are impaired may also be used.

In the present invention, as the pharmaceutical preparation containing analogues of genes, there can be mentioned, for example, a pharmaceutical preparation containing a biologically active oligonucleotide. Herein, the biologically active oligonucleotide is not particularly limited insofar as it is an oligonucleotide having a pharmaceutical effect and activity, and preferred examples include decoy, antisense oligonucleotide, aptamer, RNAi (RNA interference) effector and the like.

The term "decoy" used herein means an oligonucleotide having an activity of inhibiting transcription factor, and concrete examples of the transcription factor include NF-κB, E2F, AP-1, STAT-1, STAT-6, GATA-3, Ets and the like. Among these, NF-κB decoy oligonucleotide, E2F decoy oligonucleotide and STAT-6 decoy oligonucleotide are more preferred, and NF-κB decoy oligonucleotide is still more preferred.

As to the decoy oligonucleotide of E2F or AP-1, concrete sequences thereof are shown in WO95/11687 (SEQ ID NO:1 etc.), JP-A 6-509704, WO02/66070 and the like. As to other decoy oligonucleotides, see, for example, page 8 of WO02/066070, etc.

The antisense oligonucleotide (antisense nucleic acid) used in the present invention is not particularly limited insofar as it is a DNA or RNA that influences on expression of a specific gene, however, it usually is about 10 to 30 nucleotide long. Concrete examples of the antisense oligonucleotide include ISIS-2302, ISIS-2503, ISIS-2922, ISIS-3521, ISIS-5132, ISIS-14803, ISIS-14838, ISIS-15839, G-3139, IN-3001, GPI-2A, EPI-2010 and the like. ISIS-2302, ISIS-14838 and ISIS-15839 are more preferred. Concrete sequences of these are found in Japanese patent No. 2732546 and JP-A 2002-526125 or US Patent No. 6,096,722, etc.

Furthermore, the aptamer used in the present invention is not particularly limited insofar as it is a RNA molecule that specifically binds to a specific protein and controls the action of the specific protein. Also, the RNAi effector is not particularly limited insofar as it is a siRNA (small interfering RNA) that is a small dsRNA (double-strand RNA) causing gene silencing after transcription.

Preferred examples of the medical liquid include pharmaceutical preparations containing NF-κB decoy oligonucleotide as a main component. NF-κB decoy oligonucleotide may be oligonucleotides that specifically compete with a nucleic acid binding site of NF-κB existing on chromosome, and may be analogues of such oligonucleotides. As preferred decoy oligonucleotides of NF-κB, oligonucleotides including a base sequence gggatttccc (sequence of residues 8 to 17 from 5' end of SEQ ID NO: 3 of the sequence list) or its complementary sequence, mutants thereof, or compounds including these in molecule may be exemplified. The oligonucleotide may be DNA or RNA, and the oligonucleotide may include therein a modified nucleic acid and/or nucleic acid mimic. These oligonucleotides, mutants thereof, or compounds including these in molecule may be of single strand or double strand, and may be linear or circular. The term "mutant" used herein refers to oligonucleotides in which the above sequence is partially mutated, substituted, inserted or deleted and specifically compete with a nucleic acid binding site to which NF-κB binds. The oligonucleotide used in the present invention also includes oligonucleotides modified so that they are difficult to be decomposed in a biological body, such as oligonucleotide having a thiophosphoric acid diester bond in which oxygen atom in a phosphoric acid diester bond is substituted by a sulfur atom (s-oligo) or oligonucleotide in which a phosphoric acid bond is substituted by a methylphosphate group having no electric charge.

Pharmaceutical preparations containing NF-κB decoy oligonucleotide as a main component are effective in therapy and prophylaxis of diseases caused by NF-κB, namely, diseases caused by undesired activation of a gene controlled by the transcription factor NF-κB. To be more specific, they are effective in prophylaxis and therapy of ischemic diseases such as myocardial infarction and brain infarction, and autoimmune diseases such as rheumatism and atopy; prevention of worse prognosis after organ transplantation or surgery; and prophylaxis of carcinoma metastasis or invasion.

In the present invention, a variety of pharmaceutical preparations containing genes and/or analogues thereof other than the above pharmaceutical preparations may be used.

### EXAMPLES

The present invention will be described more specifically by way of the following examples; however, the present invention is not limited by these examples.

### [Example 1: Cellular transfection of luciferase plasmid]

### (1) Experimental method

On a skin of back of rat, 50 µg/animal or 100 µg/animal of luciferase plasmid (available from Promega Corp., pGL3E luciferase plasmid) was dosed using a normal syringe with needle (comparative) and a needleless syringe ShimaJET (ShimaJET P/N 555-15000, Shimadzu Corp.) respectively, and after 24 hours, a skin sample was collected and measured for luciferase activity using a measuring machine (Lumat LB9507, available from EG&G Berthold Technologies). The measurement was repeated three times and average and standard deviation were determined.

### (2) Results

| Group | Dose amount | Luciferase activity | (unit: RLU) |
|---|---|---|---|
| | | Average ± | Standard deviation |
| Untreated (Control) | | 21312.181 ± | 10656.090 |
| Syringe with needle | 50 µg | 11836.488 ± | 4832.226 |
| Syringe with needle | 100 µg | 38224.267 ± | 15613.157 |
| ShimaJET | 50 µg | 3111827.866 ± | 1270398.406 |
| ShimaJET | 100 µg | 3399258.233 ± | 1387741.362 |

As is apparent from the above results, when the needleless syringe ShimaJET was used, the luciferase activity was very high, revealing that the luciferase plasmid was transferred into cells with high efficiency. This demonstrates that other genes and/or analogues thereof can be transferred into cells with high efficiency.

### [Example 2: Cellular transfection of modified green fluorescent protein gene (Venus gene)]

On a skin of back of rat, Venus plasmid (100 µg/100 µL saline) was emitted by using a needleless syringe ShimaJET (ShimaJET P/N 555-15000, Shimadzu Corp.), and the skin of the emission part was collected after two days. A frozen section was created and observed under a fluorescent microscopy.

The Venus plasmid was constructed by inserting about 0.7 kb of insert gene into pCS2 vector, and the details are found in Nature Biotechnology, 20(1), 87-90, 2002.

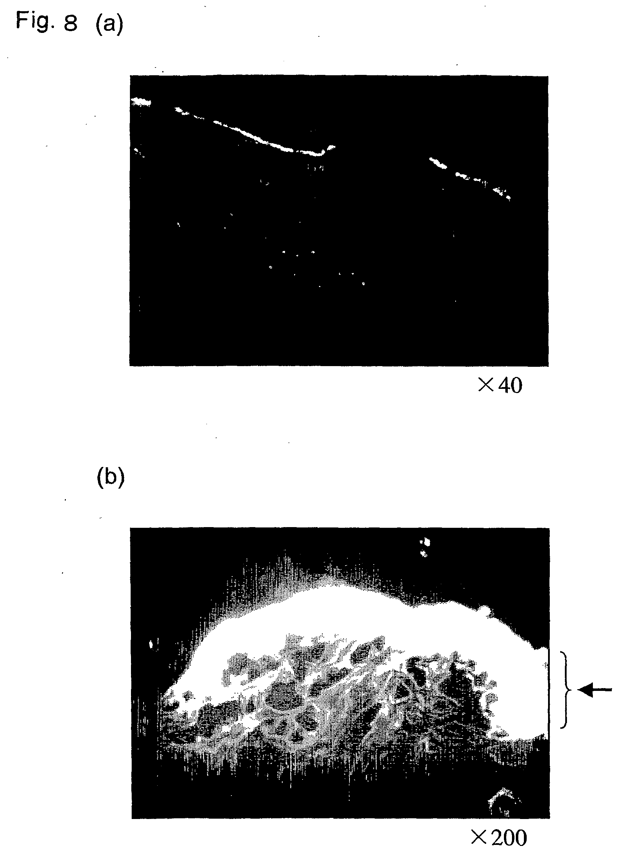

Observation results by microscopy are shown in Figs. 8. The imaging magnification is x40 for Fig. 8(a), and x200 for Fig. 8(b) (a part of Fig. 8(a) is enlarged). As is apparent from Fig.8, green fluorescence (shown by the arrow in Fig. 8(b)) was observed at granular layer in epidermal layer and transfection of the Venus gene into cell was confirmed.

### [Example 3: Cellular transfection of LacZ gene]

On a skin of back of rat, commercially available LacZ plasmid (100 µg/100 µL saline) was emitted by using a needleless syringe ShimaJET (ShimaJET P/N 555-15000, Shimadzu Corp.). After two days, the skin of the emission part was collected and the excised sample was frozen in liquid nitrogen. Thereafter, the frozen skin was fixed for 5 to 10 minutes with 1% glutalaldehyde, and washed with phosphate-buffered saline (PBS). The fixed skin was poured with a βgal staining solution, incubated at 37°C overnight, washed, and then stained with HE (hematoxylin-eosin stain) for microscopic observation.

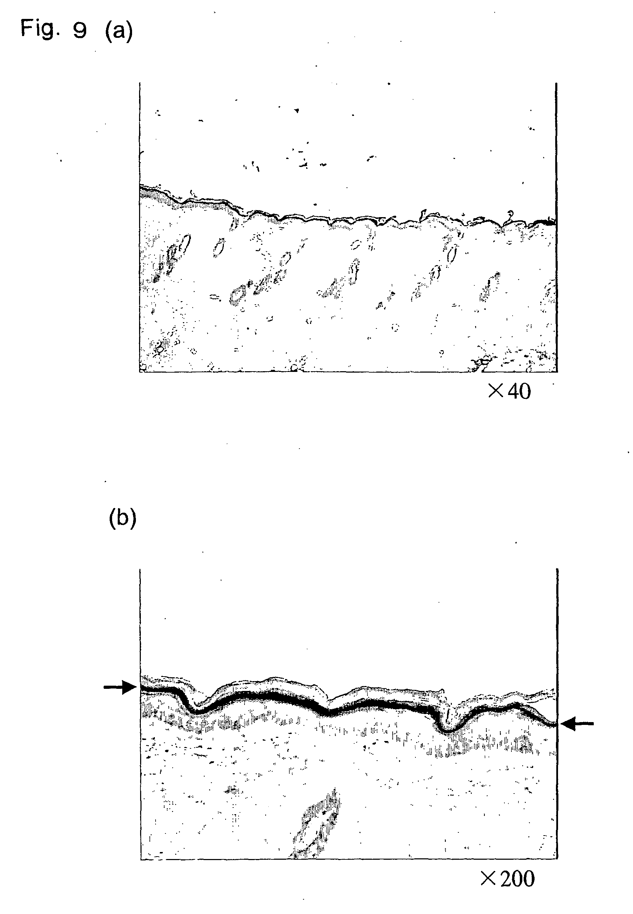

Results of microscopic observation are shown in Fig. 9. The imaging magnification is F x40 for Fig. 9(a), and x200 for Fig. 9(b) (a part of Fig. 9(a) is enlarged). As is apparent from Fig. 9, a stain-positive part (blue-stained β-galactosidase activity positive part as shown by the arrow in Fig. 9(b)) was observed at granular layer in epidermal layer and hence transfection of the LacZ gene into cell was confirmed.

As the βgal staining solution, a mixed solution of 20 µL of 50 mg/mL bromochloroindolyl-beta-D-galactopyranoside (BCIG) dimethylformamide solution, 10 µL of 0.1M MgCl₂, 10 µL of 0.5M K₃Fe(CN)₆, 10 µL of 0.5M K₄Fe(CN)₆ and 950 µL of PBS, or β-Galactosidase Staining Kit (available from Mirus International Inc.) may be used.

### [Example 4: Cellular transfection of HGF gene]

On a skin of back of rat, pVAX-HGF plasmid (CAS No.:627861-07-8, 100 µg/100 µL saline) was emitted using a ShimaJET. After two days, every layer of the skin in the emission part was separated off and washed with PBS. Then the separated skin was cut as finely as possible with scissors, and put into an extraction buffer (20 mM Tris-HCl buffer (pH 7.5), 2M NaCl, 0.1% Tween 80, 1mM EDTA, 1mM PMSF) and homogenized using a Polytron homogenizer. Then the resultant homogenate was centrifuged at 15,000 rpm at 4°C for 30 minutes, and the supernatant was collected.

This supernatant was measured for HGF concentration using a human HGF ELISA kit (available from Biosource, Inc., Catalog No. KAC2211).

For reference groups, a group using a syringe with needle and a control group were prepared. In the group using a syringe with needle, measurement of HGF concentration was conducted in the same manner as described above except that a syringe with needle was used in place of the ShimaJET, while in the control group, measurement of HGF concentration was conducted in the same manner as described above except that only saline was used in place of 100 µg/100 µL pVAX-HGF plasmid in saline.

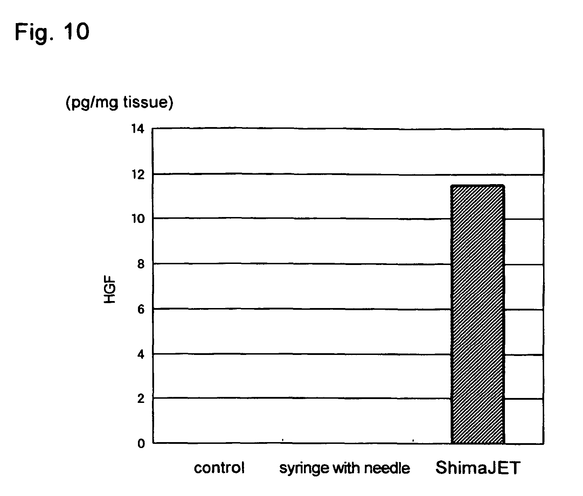

Fig. 10 shows amounts of HGF that were intradermally injected using a syringe with needle or by ShimaJET emission. In Fig. 10, the vertical axis represents an amount of HGF (pg) per 1 mg of tissue. In brief, 0 pg/mg tissue of HGF for the control group, 0 pg/mg tissue of HGF for the syringe with needle group, and 11.49 pg/mg tissue of HGF for the ShimaJET emission group were determined. This revealed that expression of HGF protein was not observed when a syringe with needle was used, whereas Cellular transfection of HGF gene was observed in the case of ShimaJET emission.

As described above, in the above examples, luciferase plasmid, Venus plasmid, LacZ plasmid and pVAX-HGF plasmid were intradermally injected using a needleless syringe, ShimaJET. However, the present invention may be applied not only to the above needleless syringe, but also any needleless syringes having a medical agent chamber and pressure application means. The present invention may be applied not only to the above medical agent, but also any pharmaceutical preparations containing genes and/or analogues thereof. Therefore, the above examples are merely exemplification in every respect, and should not be interpreted limitatively. Furthermore, any modifications within the equivalents of the claims are included in the scope of the present invention.

## Claims

1. A needleless syringe comprising:
a medical agent chamber having one or multiple nozzle bore(s) and accommodated with a medical agent, and
a syringe main body being provided with pressure application means to apply pressure on the medical agent toward a direction of the nozzle bore (s) and effect emission of the medical agent through the nozzle bore(s), wherein
the medical agent is a pharmaceutical preparation containing genes and/or analogues thereof.

2. The needleless syringe according to claim 1, wherein the pharmaceutical preparation containing genes and/or analogues thereof is in a form of a carrier retaining the genes and/or analogues thereof.

3. The needleless syringe according to claim 2, wherein the carrier is a metal particle.

4. The needleless syringe according to claim 3, wherein the metal particle is a colloidal gold particle.

5. A needleless syringe comprising:
a medical liquid chamber having one or multiple nozzle bore(s), accommodated with a medical liquid, and provided with a piston so as to be located opposite to the nozzle bore (s) across the medical liquid; and
a syringe main body being provided with a pressure application mechanism to apply pressure on the piston toward a direction of the nozzle bore (s) and effect emission of the medical liquid through the nozzle bore(s), wherein
the medical liquid is a pharmaceutical preparation containing genes and/or analogues thereof.

6. The needleless syringe according to claim 5, wherein the medical liquid is a pharmaceutical preparation containing a gene coding an angioproliferator or an angiogenesis protein.

7. The needleless syringe according to claim 6, wherein the angioproliferator or the angiogenesis protein is one selected from the group consisting of scatter factor/hepatocyte growth factor (HGF), vascular endothelial growth factor (VEGF), fibroblast growth factor (FGF), transforming growth factor (TGF), erythropoietin (EPO), angiogenin, pleiotrophin (PTN, HB-GAM), midkine, placental growth factor protein (PIGF), platelet-derived growth factor (PDGF), Del-1 (Developmentally Regulated Endothelial Cell Locus-1), angiopoietin, folistatin, granulocyte colony stimulating factor (G-CSF), leptin, insulin-like growth factor (IGF), chicken ovalbumin upstream promoter-transcription factor II (COUP-TFII), endothelial NO synthetase (eNOS), inductive NO synthetase (iNOS), human monocyte chemotactic factor (monocyte chemotactic protein-1, MCP-1), proliferin and ephrin.

8. The needleless syringe according to claim 5, wherein the medical liquid is a pharmaceutical preparation containing a gene coding HGF.

9. The needleless syringe according to claim 8, wherein the pharmaceutical preparation containing a gene coding HGF is a pharmaceutical preparation containing an expression vector into which a gene coding HGF is inserted.

10. The needleless syringe according to claim 9, wherein the expression vector is one selected from the group consisting of plasmids, adenovirus vectors and HVJ-E vectors.

11. The needleless syringe according to claim 5, wherein the medical liquid is a pharmaceutical preparation containing a biologically active oligonucleotide.

12. The needleless syringe according to clam 11, wherein the biologically active oligonucleotide is decoy, antisense oligonucleotide, aptamer or RNAi effector.

13. The needleless syringe according to claim 12, wherein the antisense oligonucleotide is one selected from the group consisting of ISIS-2302, ISIS-2503, ISIS-2922, ISIS-3521, ISIS-5132, ISIS-14803, ISIS-14838, ISIS-15839, G-3139, IN-3001, GPI-2A and EPI-2010.

14. The needleless syringe according to claim 12, wherein the decoy is a transcription factor inhibiting decoy oligonucleotide.

15. The needleless syringe according to claim 14, wherein the transcription factor is one selected from the group consisting of NF-κB, E2F, AP-1, STAT-1, STAT-6, GATA-3 and Ets.

16. The needleless syringe according to claim 5, wherein the medical liquid is a pharmaceutical preparation containing NF-κB decoy oligonucleotide.

17. The needleless syringe according to any one of claims 5 to 16, wherein the medical liquid chamber is formed inside a nozzle ampoule that is detachably attached to the syringe main body.

18. The needleless syringe according to claim 17, wherein
the nozzle ampoule has an ampoule opening in which the piston is exposed, the opening verge of the ampoule being formed with a plurality of convex fitting portions,
the syringe main body has a hollow housing accommodating the pressure application mechanism, the housing having a housing opening communicated with the ampoule opening, the opening verge of the housing being formed with concave fitting portions corresponding to the convex fitting portions provided in the nozzle ampoule, and
the convex fitting portions and the concave fitting portions are fitted or removed by rotating the nozzle ampoule and the housing in mutually opposite directions.

19. The needleless syringe according to claim 17 or 18, wherein the pressure application mechanism is provided with, a pressure application member which is movable in the direction of approaching/leaving the piston, a compression spring that biases the pressure application member toward the direction of approaching the piston, and a holding member that releasably holds movement of the pressure application member by the spring in the state that the spring is compressed.

20. The needleless syringe according to claim 19, wherein
in the housing formed are a spring chamber having a housing opening at one end of the spring chamber, and a releasing chamber partitioned by a partition wall having a through hole from the spring chamber,
the pressure application member is provided with a spring receiver located in the spring chamber and abutted against the piston, and a rod extending from the spring receiver through the through hole of the partition wall and projecting into the release chamber, an engaging projection being formed on an outer surface of projecting end of the rod in such a dimension that the engaging projection can not pass through the through hole of the partition wall, the spring being a coil spring interposed between the partition wall and the spring receiver so as to surround the rod, and
the holding member has a stopper face against which the engaging projection of the rod is abutted in the state that the spring is compressed.

21. The needleless syringe according to claim 20, wherein the holding member is provided with a ring portion received by the partition wall in the releasing chamber so as to surround the rod, and a cylindrical part provided upright on the ring portion and having a tip end face designed as the stopper face, and a plurality of slits are formed so as to longitudinally pass through the cylindrical part.

22. The needleless syringe according to claim 21, wherein opposite faces of the each slit are partially connected via a breakable portion.

23. The needleless syringe according to claim 21 or 22, wherein the housing has an end wall that opposes the partition wall while leaving a space therebetween, a releasing member is held so as to be able to approach/leave the stopper face through a holding hole formed in the end wall, the stopper face is formed into a tapered recess that spreads as the stopper face comes closer to the releasing member, and the releasing member is provided with a push-spreading face that is formed into a tapered projection corresponding to the stopper face.

24. The needleless syringe according to claim 20, wherein the holding member comprises a stopper piece that is movably inserted in a locking hole and has the stopper face in an inward end, the locking hole penetrating a peripheral wall of the releasing chamber from inside to outside thereof and extending in the direction orthogonal to the rod.

25. The needleless syringe according to claim 24, wherein on an outer surface of the peripheral wall of the releasing chamber is formed a guide groove which extends in the direction orthogonal to the locking hole and permits an outward end of the locking hole to open at a bottom face of the guide groove, a locking member is movably fitted into the guide groove, in an inner lateral surface of the locking member is formed a recess that allows insertion of an outward end of the stopper piece.

26. The needleless syringe according to any one of claims 20 to 25, wherein a safety cap which is attached to the syringe main body is provided in place of the nozzle ampoule, the safety cap having a convex fitting portion which is equivalent to the convex fitting portion of the nozzle ampoule, and a gap is kept between the engaging projection of the rod and the stopper face of the holding member in the state that the convex fitting portion of the safety cap is fitted into the concave fitting portion of the housing.

27. The needleless syringe according to any one of claims 5 to 16, wherein the medical liquid chamber is integrated with the syringe main body.

28. The needleless syringe according to claim 27, wherein the medical liquid chamber is formed inside the nozzle ampoule that is integrated with the syringe main body.

29. The needleless syringe according to claim 27 or 28, wherein
the syringe main body has a hollow housing accommodating the pressure application mechanism,
the pressure application mechanism is provided with, a pressure application member which is movable in the direction of approaching/leaving the piston, a compression spring that biases the pressure application member toward the direction of approaching the piston, and a holding member that releasably holds movement of the pressure application member by the spring in the state that the spring is compressed,
in the housing are formed a spring chamber having a housing opening at one end of the spring chamber, and a releasing chamber that is partitioned by a partition wall having a through hole from the spring chamber,
the pressure application member is provided with a spring receiver located in the spring chamber and abutted against the piston, and a rod extending from the spring receiver through the through hole of the partition wall and projecting into the release chamber, an engaging projection being formed on an outer surface of projecting end of the rod in such a dimension that the engaging projection can not pass through the through hole of the partition wall, the spring being a coil spring interposed between the partition wall and the spring receiver so as to surround the rod, and
the holding member is provided with a ring portion received by the partition wall so as to surround the rod in the releasing chamber, and a cylindrical part provided upright on the ring portion, a tip end face of the cylindrical part being designed as a stopper face against which the engaging projection of the rod is abutted in the state that the spring is compressed, a plurality of slits being formed so as to longitudinally pass through the cylindrical part.

30. The needleless syringe according to claim 29, wherein opposite faces of the each slit are partially connected via a breakable portion.

31. The needleless syringe according to claim 29 or 30, wherein the housing has an end wall that opposes the partition wall while leaving a space therebetween, a releasing member is held so as to be able to approach/leave the stopper face through a holding hole formed in the end wall, the stopper face is formed into a tapered recess that spreads as the stopper face comes closer to the releasing member, and the releasing member is provided with a push-spreading face that is formed into a tapered projection corresponding to the stopper face.

32. The needleless syringe according to claim 27 or 28, wherein
the syringe main body has a hollow housing accommodating the pressure application mechanism,
the pressure application mechanism is provided with a pressure application member which is movable in the direction of approaching/leaving the piston, a compression spring that biases the pressure application member toward the direction of approaching the piston, and a holding member that releasably holds movement of the pressure application member by the spring in the state that the spring is compressed,
in the housing are formed a spring chamber having a housing opening at one end of the spring chamber, and a releasing chamber that is partitioned by a partition wall having a through hole from the spring chamber,
the pressure application member is provided with a spring receiver located in the spring chamber and abutted against the piston, and a rod extending from the spring receiver through the through hole of the partition wall and projecting into the release chamber, an engaging projection being formed on an outer surface of projecting end of the rod in such a dimension that the engaging projection can not pass through the through hole of the partition wall, the spring being a coil spring interposed between the partition wall and the spring receiver so as to surround the rod, and
the holding member comprises a stopper piece that is movably inserted in a locking hole, the locking hole penetrating a peripheral wall of the releasing chamber from inside to outside thereof and extending in the direction orthogonal to the rod, a stopper face against which the engaging projection of the rod isabutted in the state that the spring is compressed being formed in an inward end of the stopper piece.

33. The needleless syringe according to claim 32, wherein on an outer surface of the peripheral wall of the releasing chamber is formed a guide groove which extends in the direction orthogonal to the locking hole and permits an outward end of the locking hole to open at a bottom face of the guide groove, a locking member is movably fitted into the guide groove, in an inner lateral surface of the locking member is formed a recess that allows insertion of an outward end of the stopper piece.
